(19)

**Europäisches Patentamt**
**European Patent Office**
**Office européen des brevets**

(11) **EP 4 760 253 A1**

(12) **EUROPEAN PATENT APPLICATION**

(43) Date of publication:
**17.06.2026 Bulletin 2026/25**

(21) Application number: **25217686.2**

(22) Date of filing: **21.11.2025**

(51) International Patent Classification (IPC):
**G01N 29/024** (2006.01)    **G01N 33/32** (2006.01)
**G01N 21/17** (2006.01)    **G01N 29/24** (2006.01)

(52) Cooperative Patent Classification (CPC):
**G01N 21/1702; G01N 29/024; G01N 29/2418;**
**G01N 33/32**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **10.12.2024 IN 202421097522**

(71) Applicant: **Tata Consultancy Services Limited Maharashtra (IN)**

(72) Inventors:
• **GOREY, Abhijeet**
  **700156 Kolkata (IN)**

• **GUPTA, Pathikrit**
  **700156 Kolkata (IN)**
• **DAS, Rajat Kumar**
  **700091 Kolkata (IN)**
• **BHAUMIK, Chirabrata**
  **700091 Kolkata (IN)**
• **CHAKRAVARTY, Tapas**
  **700091 Kolkata (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **SYSTEMS AND METHODS FOR PREDICTING VOLUME SOLIDS MEASUREMENT OF PAINTS BASED ON NON-INVASIVE PHOTOACOUSTIC SENSING**

(57)    Volume solids (VS) is considered to be a very important and critical property of paint in paint related industries. Conventional methods for VS measurement are usually cumbersome, time-consuming and laborious. The present disclosure resolves the problems of the conventional approaches by providing a system and method for predicting volume solids measurement of paints based on non-invasive photoacoustic sensing. The present disclosure provides a non-invasive and compact photoacoustic (PA) sensing method with artificial intelligence (AI) model to predict volume solid measurements of paint samples. In the method of the present disclosure, a time domain photoacoustic (PA) signal obtained from the paint is used to predict the volume solids measurements. A temporal shift of the time domain PA signal is used as a feature and a regression based prediction model is trained to predict volume solids measurements of the paint samples.

Receiving a plurality of wet paint samples in a transparent sample container of a predefined width and a plurality of volume solid (VS) labels associated to the plurality of wet paint samples — 202

↓

Providing an excitation signal to each of plurality of wet paint samples using a pulsed laser diode of a predefined wavelength and a predefined optical power — 204

↓

Obtaining a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples — 206

↓

Generating a second type of time domain photoacoustic (PA) signal corresponding to each of plurality of wet paint samples by performing a plurality of operations on the first type of time domain photoacoustic (PA) signal corresponding to each of plurality of wet paint samples — 208

↓

Extracting one or more features from the second type of time domain photoacoustic (PA) signal corresponding to each of plurality of wet paint samples using a signal analysis technique — 210

↓

Determining a velocity parameter of the second type of time domain photoacoustic (PA) signal corresponding to each of plurality of wet paint samples based on the one or more features and the predefined width of the transparent sample container — 212

↓

Predicting a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training — 214

200 —    **FIG. 2**

EP 4 760 253 A1

**Description**

CROSS-REFERENCE TO RELATED APPLICATIONS AND PRIORITY

The present application claims priority to Indian application no. 202421097522, filed on December 10, 2024.

TECHNICAL FIELD

The disclosure herein generally relates to the field of paint manufacturing, and, more particularly, to systems and methods for predicting volume solids measurement of paints based on non-invasive photoacoustic sensing.

BACKGROUND

Paint is a complex fluid comprising solvents, binders, additives, pigments, resin, and/or the like. Out of different rheological properties of the paint, volume solid (VS) is a very important and critical property particularly for coil coating industries and other industries including automobile paint shops, submarines, aerospace, and/or the like. The change in VS affects overall rheology of the paint including aesthetic quality of paint coatings. Apart from paint ingredients, the VS is impacted by physical conditions such as temperature, pressure and moisture. Hence, monitoring the VS in a specific time interval is of a particular importance to industries.

Conventionally, VS is measured by measuring a wet film thickness once the paint is applied and then allowing the paint to oven dry and further measuring the dry film thickness of the paint. Wet film thickness gauge is used to measure the wet film thickness whereas cross-sectional microscopy is used to measure the dry film thickness of the paint layer. Alternatively, a method is proposed to measure VS by measuring weight of wet paint, its specific gravity and dry film thickness of a particular area. Since these methods measure dry film thickness, it becomes time consuming as oven drying of the paint needs a specific time. In current practices, a small portion of a sample is painted to measure the VS and if the desired value of the VS is achieved then the overall sample is painted. Additionally, certain modifications to the wet paint or painting process are made if the desired VS is not achieved. These methods are usually cumbersome, time-consuming and laborious.

SUMMARY

Embodiments of the present disclosure present technological improvements as solutions to one or more of the above-mentioned technical problems recognized by the inventors in conventional systems. For example, in one embodiment, a processor implemented method is provided. The processor implemented method, comprising: receiving, via one or more hardware processors, a plurality of wet paint samples in a transparent sample container of a predefined width and a plurality of volume solid (VS) labels associated to the plurality of wet paint samples; providing, via the one or more hardware processors, an excitation signal to each of plurality of wet paint samples using a pulsed laser diode of a predefined wavelength and a predefined optical power; obtaining, via the one or more hardware processors, a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples; generating, via the one or more hardware processors, a second type of time domain photoacoustic (PA) signal corresponding to each of plurality of wet paint samples by performing a plurality of operations on the first type of time domain photoacoustic (PA) signal corresponding to each of plurality of wet paint samples; extracting, via the one or more hardware processors, one or more features from the second type of time domain photoacoustic (PA) signal corresponding to each of plurality of wet paint samples using a signal analysis technique; determining, via the one or more hardware processors, a velocity parameter of the second type of time domain photoacoustic (PA) signal corresponding to each of plurality of wet paint samples based on the one or more features and the predefined width of the transparent sample container; and predicting, via the one or more hardware processors, a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training.

In another aspect, a system is provided. The system comprising a memory storing instructions; one or more Input/Output (I/O) interfaces; and one or more hardware processors coupled to the memory via the one or more I/O interfaces, wherein the one or more hardware processors (104) are configured by the instructions to: receive a plurality of wet paint samples in a transparent sample container of a predefined width and a plurality of volume solid (VS) labels associated to the plurality of wet paint samples; provide an excitation signal to each of plurality of wet paint samples using a pulsed laser diode of a predefined wavelength and a predefined optical power; obtain a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples; generate a second type of time domain photoacoustic (PA) signal corresponding to each of plurality of wet paint samples by performing a plurality of operations on the first type of time domain photoacoustic (PA) signal corresponding to each of plurality of wet paint samples; extract one or more features from the second type of time domain photoacoustic (PA) signal corresponding to each of plurality of wet paint samples using a signal analysis technique; determine a velocity parameter of the second type of time domain photoacoustic (PA) signal corresponding to each of plurality of wet paint samples based on the one or more features and the predefined width of the transparent sample container; predict a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training.

In yet another aspect, a non-transitory computer readable medium is provided. The non-transitory computer readable medium are configured by instructions for providing receiving a plurality of wet paint samples in a transparent sample container of a predefined width and a plurality of volume solid (VS) labels associated to the plurality of wet paint samples; providing an excitation signal to each of plurality of wet paint samples using a pulsed laser diode of a predefined wavelength and a predefined optical power; obtaining a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples; generating a second type of time domain photoacoustic (PA) signal corresponding to each of plurality of wet paint samples by performing a plurality of operations on the first type of time domain photoacoustic (PA) signal corresponding to each of plurality of wet paint samples; extracting one or more features from the second type of time domain photoacoustic (PA) signal corresponding to each of plurality of wet paint samples using a signal analysis technique; determining a velocity parameter of the second type of time domain photoacoustic (PA) signal corresponding to each of plurality of wet paint samples based on the one or more features and the predefined width of the transparent sample container; and predicting a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training.

In accordance with an embodiment of the present disclosure, the predefined wavelength of the pulse laser diode is selected based on a color criterion of each of the plurality of wet paint samples.

In accordance with an embodiment of the present disclosure, the predefined optical power of the pulse laser diode is selected based on one or more characteristics of the transparent sample container and a type of each of the plurality of wet paint samples.

In accordance with an embodiment of the present disclosure, the first type of time domain photoacoustic (PA) signal represents a raw time domain photoacoustic (PA) signal.

In accordance with an embodiment of the present disclosure, the second type of time domain photoacoustic (PA) signal represents a digitized time domain photoacoustic (PA) signal.

In accordance with an embodiment of the present disclosure, the plurality of operations performed on the first type of time domain photoacoustic (PA) signal includes at least one of (i) signal and nose ratio (SNR) enhancement, (ii) signal averaging, (iii) signal amplification, and (iv) signal digitization.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for predicting volume solids measurement of paints based on non-invasive photoacoustic sensing, in accordance with some embodiments of the present disclosure.

FIG. 2 illustrates an exemplary flow diagram illustrating a method for predicting volume solids measurement of paints based on non-invasive photoacoustic sensing, using the system of FIG. 1, in accordance with some embodiments of the present disclosure.

FIG. 3 depicts a block diagram representing a photoacoustic experimental setup for predicting volume solids measurement of paints based on non-invasive photoacoustic sensing, in accordance with some embodiments of the present disclosure.

FIGS. 4A through 4D depict graphical representations illustrating experimental results for predicting volume solids measurement of paints based on non-invasive photoacoustic sensing, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

Paint is a complex fluid comprising solvents, binders, additives, pigments, resin, and/or the like. Out of different rheological properties of the paint, volume solids (VS) is a very important and critical property particularly for coil coating industries and other industries including automobile paint shops, submarines, aerospace, and/or the like. The change in VS affects overall rheology of the paint including aesthetic quality of paint coatings. Apart from paint ingredients, the VS is impacted by physical conditions such as temperature, pressure and moisture. Hence, monitoring the VS in a specific time interval is of a particular importance to industries. Physically, VS is a measure of total volume of the solid materials in a given volume of wet paint, expressed in percentage. VS is given as shown in equation (1) below as:

$$\text{VS} = \frac{\text{volume of binder} + \text{volume of pigment}}{\text{Total volume}} \times 100 \qquad (1)$$

The change in VS affects overall rheology of the paint including aesthetic quality of paint coatings. The VS is impacted by physical conditions such as temperature, pressure and moisture. Hence, monitoring the VS in a specific time interval is of a particular importance to industries. Conventionally, VS is measured by measuring a wet film thickness once the paint is applied and then allowing the paint to oven dry and further measuring the dry film thickness of the paint. Wet film thickness gauge is used to measure the wet film thickness whereas cross-sectional microscopy is used to measure the dry film thickness of the paint layer. These methods are usually cumbersome, time-consuming and laborious. Alternatively, a method is proposed to measure VS by measuring weight of the wet paint, its specific gravity and dry film thickness of a particular area. With this method VS is given as shown in equation (2) below:

$$VS = \frac{\text{A.t.}\rho}{\text{W}} \qquad (2)$$

Here, A represents area of the dry film thickness, $t$ represents the dry film thickness, $\rho$ represents density of the wet paint and $W$ represents the weight of the wet paint. Since these methods measure the dry film thickness, it becomes time consuming as the oven drying of the paint needs a specific time. In current practices, a small portion of a sample is painted to measure the VS and if the desired value of VS is achieved then the overall sample is painted. Additionally, certain modifications to the wet paint or painting process are made if the desired VS is not achieved. Thus, there is no sensor available to measure the VS of a paint from the wet paint.

Embodiments of the present disclosure resolve the problems of the conventional approaches by providing a system and method for predicting volume solids measurement of paints based on non-invasive photoacoustic sensing. The present disclosure provides a compact, non-invasive and cost-effective, photoacoustic sensor combined with artificial intelligence (AI) for measuring the VS from a wet paint. The photoacoustic (PA) is a hybrid sensing modality comprising of optical excitation and the acoustic acquisition. In PA, optical laser pulses of a particular wavelength and time-width are used to irradiate a sample. The sample absorbs the optical laser pulses and undergoes thermoelastic expansion followed by contraction to generate acoustic or PA waves. These time domain PA waves are processed and analyzed to determine properties of different materials in solid, liquid or gases states. In the present disclosure, paint samples are irradiated with the optical laser pulses to obtain the PA waves. From the paint samples, the PA waves can depict its optical as well as mechanical properties as it involves acoustic waves. Equations (1) and (2) suggest that the VS of a wet paint is directly proportional to the density of the paint. Hence, estimating the density of the PA wave can help to predict the VS of the wet paint. For quantitatively measuring the VS through a PA sensor, the method of the present disclosure adapts a regression-based AI model to predict the VS of the paint samples. Due to limitation of paint samples with actual ground truth of VS, a total of 16 samples with 12 samples for training a model and remaining 4 samples for testing are used. A residual analysis is performed to ensure that there is no overfitting to the model.

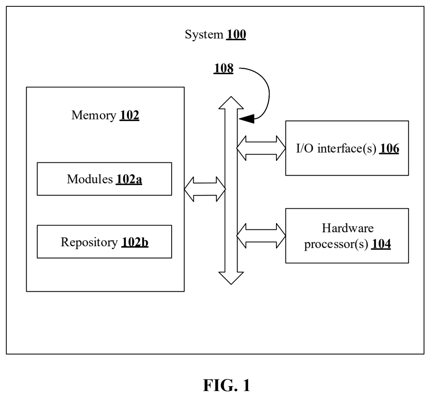

Referring now to the drawings, and more particularly to FIGS. 1 through 4D, where similar reference characters denote corresponding features consistently throughout the figures, there are shown preferred embodiments, and these embodiments are described in the context of the following exemplary system and/or method.

FIG. 1 illustrates an exemplary block diagram of a system for predicting volume solids measurement of paints based on non-invasive photoacoustic sensing, in accordance with some embodiments of the present disclosure.

In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface, and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computer, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, new data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

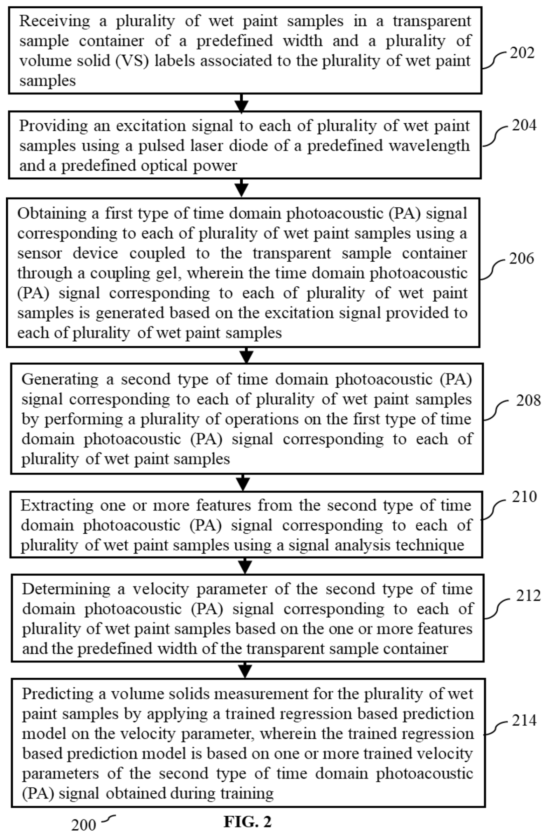

FIG. 2 illustrates an exemplary flow diagram illustrating a method for generation of prompts for predicting volume solids measurement of paints based on non-invasive photoacoustic sensing, using the system 100 of FIG. 1, in accordance with some embodiments of the present disclosure.

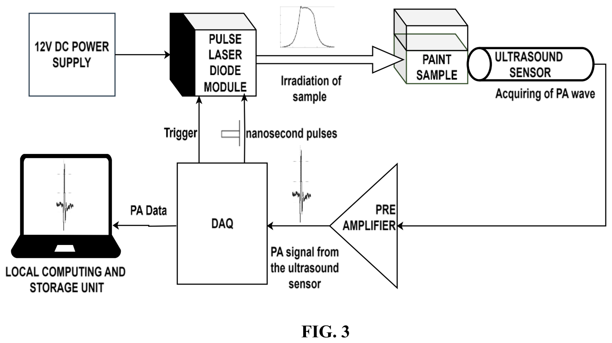

Referring to FIG. 2, in an embodiment, the system 100 comprises one or more data storage devices or the memory 102 operatively coupled to the one or more hardware processors 104 and is configured to store instructions for execution of steps of the method by the one or more processors 104. The steps of the method 200 of the present disclosure will now be explained with reference to components of the system 100 of FIG. 1, the flow diagram as depicted in FIG. 2, the block diagram of FIG. 3, and one or more examples. Although steps of the method 200 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

FIG. 3 depicts a block diagram representing a photoacoustic experimental setup for predicting volume solids measurement of paints based on non-invasive photoacoustic sensing, in accordance with some embodiments of the present disclosure.

In an embodiment, at step 202 of the present disclosure, the one or more hardware processors 104 are configured to receive a plurality of wet paint samples in a transparent sample container of a predefined width and a plurality of volume solid (VS) labels associated to the plurality of wet paint samples. As shown in FIG. 3, the paint samples are placed in the transparent sample container. The predefined width of the transparent sample container is configurable with a range of 1cm to 5cm. In the context of the present discourse, the predefined width of the transparent sample container is kept 4.2cm. Even though the predefined width of the transparent sample container is configurable, yet it is important to keep the predefined width constant for training and testing phase. Further, at step 204 of the present disclosure, the one or more hardware processors 104 are configured to provide an excitation signal to each of plurality of wet paint samples using a pulsed laser diode of a predefined wavelength and a predefined optical power. As shown in FIG. 3, the pulsed laser diode module is used for irradiating the plurality of wet paint samples. The excitation signal is an optical signal or laser pulses. In an embodiment, the laser pulse of pulse width 400ns at a peak power of 1W and beam width of 1mm is used for irradiation. The predefined optical power of the pulse laser diode is selected based on one or more characteristics of the transparent sample container and a type of each of the plurality of wet paint samples. The type of each of the plurality of wet paint samples is solvent-based, water-based, and a combination thereof. The predefined wavelength of the pulse laser diode is selected based on a color criterion of each of the plurality of wet paint samples. The plurality of wet paint samples are placed in the transparent container for the laser excitation.

Furthermore, at step 206 of the present disclosure, the one or more hardware processors 104 are configured to obtain a first type of time domain photoacoustic (PA) signal corresponding to each of plurality of wet paint samples using a sensor device coupled to the transparent sample container through a coupling gel. The time domain photoacoustic (PA) signal corresponding to each of plurality of wet paint samples is generated based on the excitation signal provided to each of plurality of wet paint samples. The first type of time domain photoacoustic (PA) signal represents a raw time domain photoacoustic (PA) signal. The generated first type of time domain PA waves within the plurality of wet paint samples are propagated through the wet paint sample and acquired by the sensor device (e.g., an ultrasound sensor). The ultrasound sensor is placed in contact with the transparent sample container through the coupling gel.

At step 208 of the present disclosure, the one or more hardware processors 104 are configured to generate a second type of time domain photoacoustic (PA) signal corresponding to each of plurality of wet paint samples by performing a plurality of operations on the first type of time domain photoacoustic (PA) signal corresponding to each of plurality of wet paint samples. The plurality of operations performed on the first type of time domain photoacoustic (PA) signal includes at least one of (i) signal and nose ratio (SNR) enhancement, (ii) signal averaging, (iii) signal amplification, and (iv) signal digitization. A pulse repetition frequency of 1 kHz with a real time averaging of 200 times is applied to enhance the SNR of acquired PA signal. Due to low SNR of averaged PA signal, it is amplified through a custom-built preamplifier at a gain of 60dB. Further, an Analog Discovery-2 (AD2) is used as a data acquisition (DAQ) system to digitize and transfer the PA signal to a processor for signal processing and analysis. Also, the AD2 is used to trigger the laser pulses and synchronize data acquisition. For an entire set of experiments, physical conditions, laser power and position of the pulse laser diode and ultrasound sensor is kept fixed. The second type of time domain photoacoustic (PA) signal is obtained as the output. The second type of time domain photoacoustic (PA) signal represents a digitized time domain photoacoustic (PA) signal.

Further, at step 210 of the present disclosure, the one or more hardware processors 104 are configured to extract one or more features from the second type of time domain photoacoustic (PA) signal corresponding to each of plurality of wet paint samples using a signal analysis technique. The one or more features may include but are not limited to variation of peak time exhibiting a temporal shift and velocity of the second type of time domain photoacoustic (PA) signal.

At step 212 of the present disclosure, the one or more hardware processors 104 are configured to determine a velocity parameter of the second type of time domain photoacoustic (PA) signal corresponding to each of plurality of wet paint samples based on the one or more features and the predefined width of the transparent sample container. The velocity parameter of the second type of time domain photoacoustic (PA) signal representing digitized time domain PA signal is determined using the one or more extracted features such as peak time of the digitized time domain PA signal and the width of the transparent sample container.

At step 214 of the present disclosure, the one or more hardware processors 104 are configured to predict a volume solids measurement for the plurality of wet paint samples by applying a trained regression based prediction model on the velocity parameter. The trained regression based prediction model is based on one or more trained velocity parameters of the second type of time domain photoacoustic (PA) signal obtained during training..

In an embodiment, the step 210 through 216 are further described by way of following exemplary explanation.

In PA, irradiation of a nano-second (ns) laser pulse results in localized temperature excursion within a wet paint sample leading to a transient pressure rise and thermoelastic expansion. Thus, PA source act as a strain source. Since paint is a highly heterogeneous visco-elastic fluid, a spatial variation in pressure wave induces the PA waves of wide frequency range at an irradiation point. In an embodiment, wide range of frequencies in the PA waves can be attributed to narrow time width PA signal. These PA waves propagate through a medium by momentum transfer and are represented by the following equation (3):

$$\left(\nabla^2 - \frac{\rho}{k}\frac{\partial^2}{\partial t^2}\right) P(r, t) \;=\; -\frac{\beta}{C_p}\frac{\delta Q(r,t)}{\delta t} \quad (3)$$

Here, $P(r, t)$ stands for time domain PA pressure wave, $Q(r, t)$ denotes a laser enabled heating function, $\rho$ is density of the medium, $\beta$ is a thermal expansion coefficient, $k$ is the wave number and $C_p$ is specific heat at constant pressure for a given medium. Equation (3) reveals that the change in density can be observed from the time domain PA signal. Thus, equations (1), (2) and (3) theoretically signifies that the VS can be depicted from the time domain PA wave.

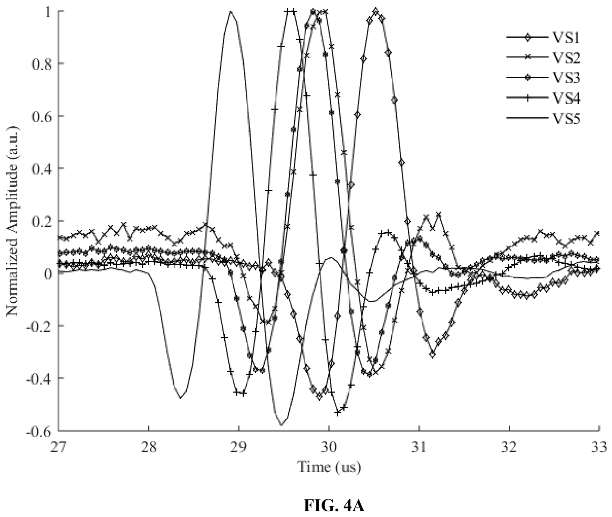

FIGS. 4A through 4D depict graphical representations illustrating experimental results for predicting volume solids measurement of paints based on non-invasive photoacoustic sensing, according to some embodiments of the present disclosure. FIG. 4A depicts a graphical representation illustrating a time domain PA signal for five wet paint samples exhibiting different VS values, according to some embodiments of the present disclosure. Since the amplitude of PA signal is dependent on color of pigment of the wet paint samples, the PA signal is normalized with respect to its peak amplitude. An identical set of containers are used for experiments and thus the temporal shift of the PA signal is due to the change in the wet paint sample's property. The wet paint samples used in the experiments are measured for their VS using the conventional methods. Therefore, a shift in the peak time (i.e., time corresponding to peak amplitude) corresponding to the VS is observed and shown in FIG. 4A.

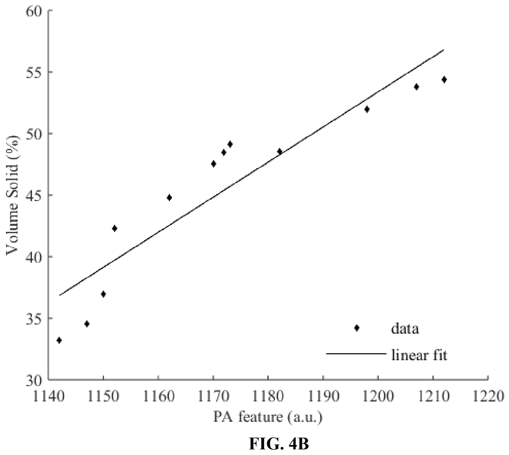

In FIG. 4A, the shift in peak time is due to a change in velocity of the PA wave propagating through the paint medium. The change in density (VS) results in the change in velocity of the PA waves. Thus, the method of the present disclosure adapts the peak time as a feature of VS of a paint. For representation purpose only 5 PA signals from a plurality of PA signals are shown in FIG. 4A. Further, a total of 16 paint samples with different VS are used in the present disclosure. 75% (i.e., 12 samples) of the total samples are used to train the model and remaining 25% (4 samples) are used to test the model. FIG. 4B depicts a graphical representation illustrating variation of peak time of PA signal with VS, according to some embodiments of the present disclosure. FIG. 4B shows the regression based prediction model with linear fitting. Accuracy (i.e., goodness of fit) of the regression based prediction model is obtained to be 87.8%. The regression based prediction model is used to measure the VS of the paint from the peak time of the PA signal. The regression based prediction model is shown in equation (4):

$$VS \;=\; |(a * m) - b| \quad (4)$$

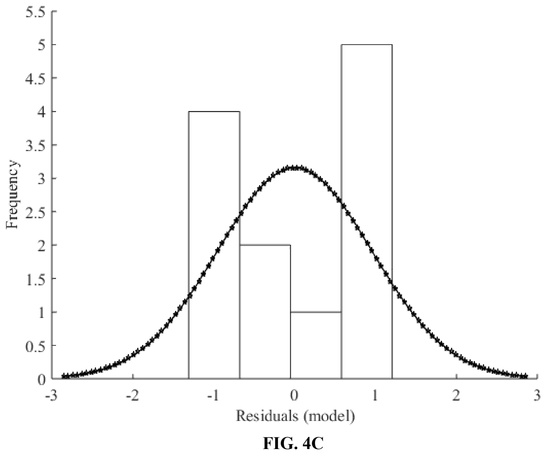

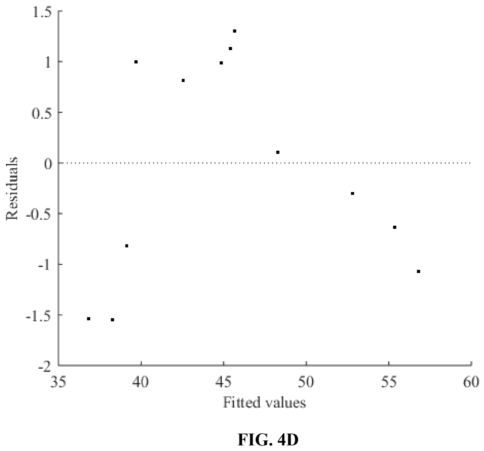

Here, $a$ and $b$ represent slope of regression curve and intercept respectively, and $m$ represents the peak time of the PA signal. In the present disclosure, values of $a$ and $b$ are 0.285 and 288.6 respectively. Since VS is a dimensionless quantity, $a$ and $b$ in equation (4) do not possess any dimensions. The accuracy of the regression based prediction model can be further enhanced by increasing sample size. Due to the limited paint samples with VS ground truth, the regression based prediction model is validated for its overfitting and efficiency. A four-fold cross validation is performed with 16 samples data of total dataset and a mean error is found to be approximately 10%. FIGS. 4C and 4D depict residual plots for predicting volume solids measurement of paints based on non-invasive photoacoustic sensing, according to some embodiments of the present disclosure. A symmetrical distribution of a probability density function over the predicted VS measurements shows that the regression based prediction model is efficient. Random distribution in FIG. 4C validated no overfitting in the regression based prediction model. Table 1 below shows the predicted VS measurements for four samples used for testing and an error in their measurements with respect to a ground truth.

Table 1

| Sample No. | Volume Solids (%) | | Error (%) |
|:---:|:---:|:---:|:---:|
| | PA Sensing | Conventional method | |
| S1 | 36.13 | 40.42 | 10.61 |
| S2 | 51.22 | 46.72 | 11.77 |
| S3 | 53.13 | 49.31 | 7.74 |

(continued)

| Sample No. | Volume Solids (%) | | Error (%) |
|---|---|---|---|
| | PA Sensing | Conventional method | |
| S4 | 42.88 | 52.46 | 18.2 |

The error is calculated using the following equation (5):

$$Error\ (\%) = \left| \frac{(Measured\ value - True\ value)}{True\ value} \right| \times 100 \quad (5)$$

The error can be mitigated through rigorous experimentation involving different types of paints with distinct values of VS. Identifying certain more features can help to enhance the accuracy of the measurements.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the embodiments or if they include equivalent elements with insubstantial differences from the literal language of the embodiments described herein.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g., hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated herein by the following claims.

**Claims**

1. A processor implemented method, comprising:

receiving (202), via one or more hardware processors, a plurality of wet paint samples in a transparent sample container of a predefined width and a plurality of volume solid (VS) labels associated to the plurality of wet paint samples;

providing (204), via the one or more hardware processors, an excitation signal to each of plurality of wet paint samples using a pulsed laser diode of a predefined wavelength and a predefined optical power;

obtaining (206), via the one or more hardware processors, a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples;

generating (208), via the one or more hardware processors, a second type of time domain photoacoustic (PA) signal corresponding to each of plurality of wet paint samples by performing a plurality of operations on the first type of time domain photoacoustic (PA) signal corresponding to each of plurality of wet paint samples;

extracting (210), via the one or more hardware processors, one or more features from the second type of time domain photoacoustic (PA) signal corresponding to each of plurality of wet paint samples using a signal analysis technique;

determining (212), via the one or more hardware processors, a velocity parameter of the second type of time domain photoacoustic (PA) signal corresponding to each of plurality of wet paint samples based on the one or more features and the predefined width of the transparent sample container; and

predicting (214), via the one or more hardware processors, a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training..

2. The processor implemented method as claimed in claim 1, wherein the predefined wavelength of the pulse laser diode is selected based on a color criterion of each of the plurality of wet paint samples.

3. The processor implemented method as claimed in claim 1, wherein the predefined optical power of the pulse laser diode is selected based on one or more characteristics of the transparent sample container and a type of each of the plurality of wet paint samples.

4. The processor implemented method as claimed in claim 1, wherein the first type of time domain photoacoustic (PA) signal represents a raw time domain photoacoustic (PA) signal.

5. The processor implemented method as claimed in claim 1, wherein the second type of time domain photoacoustic (PA) signal represents a digitized time domain photoacoustic (PA) signal.

6. The processor implemented method as claimed in claim 1, wherein the plurality of operations performed on the first type of time domain photoacoustic (PA) signal includes at least one of (i) signal and nose ratio (SNR) enhancement, (ii) signal averaging, (iii) signal amplification, and (iv) signal digitization.

7. A system (100) comprising

a memory (102) storing instructions;
one or more Input/Output (I/O) interfaces (106); and
one or more hardware processors (104) coupled to the memory (102) via the one or more I/O interfaces (106), wherein the one or more hardware processors (104) are configured by the instructions to:

receive a plurality of wet paint samples in a transparent sample container of a predefined width and a plurality of volume solid (VS) labels associated to the plurality of wet paint samples;

provide an excitation signal to each of plurality of wet paint samples using a pulsed laser diode of a predefined wavelength and a predefined optical power;

obtain a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples;

generate a second type of time domain photoacoustic (PA) signal corresponding to each of plurality of wet paint samples by performing a plurality of operations on the first type of time domain photoacoustic (PA) signal corresponding to each of plurality of wet paint samples;

extract one or more features from the second type of time domain photoacoustic (PA) signal corresponding to each of plurality of wet paint samples using a signal analysis technique;

determine a velocity parameter of the second type of time domain photoacoustic (PA) signal corresponding to each of plurality of wet paint samples based on the one or more features and the predefined width of the transparent sample container; and

predict a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training.

8. The system as claimed in claim 7, wherein the predefined wavelength of the pulse laser diode is selected based on a color criterion of each of the plurality of wet paint samples.

9. The system as claimed in claim 7, wherein the predefined optical power of the pulse laser diode is selected based on one or more characteristics of the transparent sample container and a type of each of the plurality of wet paint samples.

10. The system as claimed in claim 7, wherein the first type of time domain photoacoustic (PA) signal represents a raw time domain photoacoustic (PA) signal.

11. The system as claimed in claim 7, wherein the second type of time domain photoacoustic (PA) signal represents a digitized time domain photoacoustic (PA) signal.

12. The system as claimed in claim 7, wherein the plurality of operations performed on the first type of time domain photoacoustic (PA) signal includes at least one of (i) signal and nose ratio (SNR) enhancement, (ii) signal averaging, (iii) signal amplification, and (iv) signal digitization.

13. One or more non-transitory machine-readable information storage mediums comprising one or more instructions which when executed by one or more hardware processors cause:

receiving a plurality of wet paint samples in a transparent sample container of a predefined width and a plurality of volume solid (VS) labels associated to the plurality of wet paint samples;

providing an excitation signal to each of plurality of wet paint samples using a pulsed laser diode of a predefined wavelength and a predefined optical power;

obtaining a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples;

generating second type of time domain photoacoustic (PA) signal corresponding to each of plurality of wet paint samples by performing a plurality of operations on the first type of time domain photoacoustic (PA) signal corresponding to each of plurality of wet paint samples;

extracting one or more features from the second type of time domain photoacoustic (PA) signal corresponding to each of plurality of wet paint samples using a signal analysis technique;

determining a velocity parameter of the second type of time domain photoacoustic (PA) signal corresponding to each of plurality of wet paint samples based on the one or more features and the predefined width of the transparent sample container; and

predicting a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training.

14. The one or more non-transitory machine-readable information storage mediums as claimed in claim 13, wherein the predefined wavelength of the pulse laser diode is selected based on a color criterion of each of the plurality of wet paint samples.

15. The one or more non-transitory machine-readable information storage mediums as claimed in claim 13, wherein the predefined optical power of the pulse laser diode is selected based on one or more characteristics of the transparent sample container and a type of each of the plurality of wet paint samples, wherein the first type of time domain photoacoustic (PA) signal represents a raw time domain photoacoustic (PA) signal, wherein the second type of time domain photoacoustic (PA) signal represents a digitized time domain photoacoustic (PA) signal, and wherein the plurality of operations performed on the first type of time domain photoacoustic (PA) signal includes at least one of (i) signal and nose ratio (SNR) enhancement, (ii) signal averaging, (iii) signal amplification, and (iv) signal digitization.

System **100**

**108**

Memory **102**

Modules **102a**

Repository **102b**

I/O interface(s) **106**

Hardware processor(s) **104**

**FIG. 1**

Receiving a plurality of wet paint samples in a transparent sample container of a predefined width and a plurality of volume solid (VS) labels associated to the plurality of wet paint samples — 202

Providing an excitation signal to each of plurality of wet paint samples using a pulsed laser diode of a predefined wavelength and a predefined optical power — 204

Obtaining a first type of time domain photoacoustic (PA) signal corresponding to each of plurality of wet paint samples using a sensor device coupled to the transparent sample container through a coupling gel, wherein the time domain photoacoustic (PA) signal corresponding to each of plurality of wet paint samples is generated based on the excitation signal provided to each of plurality of wet paint samples — 206

Generating a second type of time domain photoacoustic (PA) signal corresponding to each of plurality of wet paint samples by performing a plurality of operations on the first type of time domain photoacoustic (PA) signal corresponding to each of plurality of wet paint samples — 208

Extracting one or more features from the second type of time domain photoacoustic (PA) signal corresponding to each of plurality of wet paint samples using a signal analysis technique — 210

Determining a velocity parameter of the second type of time domain photoacoustic (PA) signal corresponding to each of plurality of wet paint samples based on the one or more features and the predefined width of the transparent sample container — 212

Predicting a volume solids measurement for the plurality of wet paint samples by applying a trained regression based prediction model on the velocity parameter, wherein the trained regression based prediction model is based on one or more trained velocity parameters of the second type of time domain photoacoustic (PA) signal obtained during training — 214

200 ⌐            **FIG. 2**

**FIG. 3**

**FIG. 4A**

**FIG. 4B**

**FIG. 4C**

**FIG. 4D**

Europäisches
Patentamt

European
Patent Office

Office européen
des brevets

## EUROPEAN SEARCH REPORT

**Application Number**

EP 25 21 7686

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 983 270 A (KIKUTA MAKOTO [JP] ET AL) 8 January 1991 (1991-01-08) * column 3, lines 18-39 * | 1-15 | INV. G01N29/024 G01N33/32 G01N21/17 |
| A | JP H01 263297 A (KANSAI PAINT CO LTD) 19 October 1989 (1989-10-19) * figure 2 * | 1-15 | G01N29/24 |
| A | JP H05 52822 A (KANSAI PAINT CO LTD) 2 March 1993 (1993-03-02) * paragraphs [0015], [0020] * | 1-15 | |
| A | GOREY ABHIJEET ET AL: "A Compact Photoacoustic Sensing System for Expeditive Estimation of Paint's Viscosity", 2023 16TH INTERNATIONAL CONFERENCE ON SENSING TECHNOLOGY (ICST) IEEE, 17 December 2023 (2023-12-17), pages 1-5, XP034568540, DOI: 10.1109/ICST59744.2023.10460824 [retrieved on 2024-03-19] * figure 2 * | 1-15 | |
| A | US 2023/152212 A1 (GOREY ABHIJEET [IN] ET AL) 18 May 2023 (2023-05-18) * paragraph [0095]; figure 1 * | 1-15 | |

**TECHNICAL FIELDS SEARCHED (IPC)**

G01N

The present search report has been drawn up for all claims

| Place of search | Date of completion of the search | Examiner |
|---|---|---|
| The Hague | 1 April 2026 | van Lith, Joris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 7686

This annex lists the patent family members relating to the patent documents cited in  the above-mentioned European search report.
The members are as contained in the European Patent Office EDP file on
The European Patent Office is in no way liable for these particulars which are merely given for the purpose of information.

01-04-2026

| Patent document cited in search report | | Publication date | Patent family member(s) | | Publication date |
|---|---|---|---|---|---|
| US 4983270 | A | 08-01-1991 | JP | S6396296 A | 27-04-1988 |
| | | | US | 4983270 A | 08-01-1991 |
| JP H01263297 | A | 19-10-1989 | NONE | | |
| JP H0552822 | A | 02-03-1993 | NONE | | |
| US 2023152212 | A1 | 18-05-2023 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421097522